# EUROPÄISCHE PATENTANMELDUNG

(11) **EP 2 703 155 A1**
(43) Veröffentlichungstag der Anmeldung: **05.03.2014**
(21) Anmeldenummer: 13180306.6
(22) Anmeldetag: 13.08.2013
(51) Int. Cl.: B32B 15/085, B32B 15/20, B32B 27/32, A61M 25/00, B65D 75/12, B65D 75/58

(54) **Geräuscharme Flow-Pack-Verpackung, insbesondere zur Verpackung medizinischer Geräte**

(30) Priorität: 28.08.2012 DE 102012215270; 07.09.2012 DE 102012215937; 13.02.2013 DE 102013202321
(71) Anmelder: Huhtamaki Flexible Packaging Germany GmbH & Co. KG, 87671 Ronsberg/Allgäu (DE)
(72) Erfinder: Shaw Warren, 87487 Wiggensbach (DE); Falkowski Tomasz, 87724 Ottobeuren (DE); Fackler Tobias, 87758 Illerbeuren (DE)
(74) Vertreter: Trossin, Hans-Jürgen

(57) **Zusammenfassung**

Die vorliegende Erfindung betrifft Flow-Pack-Verpackung (30) aus einem Fo-lienlaminatstück, insbesondere zur Verpackung von medizinischen Geräten, bevorzugt von Kathetern, mit einer in Längsrichtung (L) verlaufenden Längssiegelnaht, welche entgegengesetzte Ränder des Folienlaminatstücks zu einem Folienlaminatschlauch verbindet, und mit je einer in einer zur Längsrichtung (L) orthogonalen Querrichtung (Q) verlaufenden Quersiegelnaht (32, 34) an jedem ihrer Längsenden (30a, 30b), welche Quersiegelnähte (32, 34) den Folienlaminatschlauch an seinen beiden Längsenden (30a, 30b) unter Bildung eines in Längsrichtung (L) zwischen den Quersiegelnähten (32, 34) gelegenen, vom Folienlaminatschlauch umgebenen Verpackungsraums (36) verschließen, wobei das Folienlaminat (10) eine bedruckbare äußere Lage (20), eine Barrierelage (16) und eine heißsiegelfähige Innenlage (12) umfasst, wobei die Flow-Pack-Verpackung (30) zu einem Öffnen durch Aufreißen in Längsrichtung (L) längs einer vorbestimmten Aufreißbahn ausgebildet ist, wobei die äußere Lage (20) aus monoaxial orientiertem Polyolefin, insbesondere monoaxial orientiertem Polypropylen, dessen Orientierungsrichtung (O) in Längsrichtung (L) der Verpackung (30) verläuft, gebildet ist und die siegelfähige Innenlage (12) Polyethylen umfasst, vorzugsweise aus Polyethylen gebildet ist.

## Beschreibung

Die vorliegende Erfindung betrifft eine Flow-Pack-Verpackung aus einem Folienlaminatstück, insbesondere zur Verpackung von medizinischen Geräten, bevorzugt von Kathetern, mit einer in Längsrichtung verlaufenden Längssiegelnaht, welche entgegengesetzte Ränder des Folienlaminatstücks zu einem Folienlaminatschlauch verbindet, und mit je einer in einer zur Längsrichtung orthogonalen Querrichtung verlaufenden Quersiegelnaht an jedem ihrer Längsenden, welche Quersiegelnähte den Folienlaminatschlauch an seinen beiden Längsenden unter Bildung eines in Längsrichtung zwischen den Quersiegelnähten gelegenen, vom Folienlaminatschlauch umgebenen Verpackungsraums verschließen, wobei das Folienlaminat eine bedruckbare äußere Lage, eine Barrierelage und eine heißsiegelfähige Innenlage umfasst, wobei die Flow-Pack-Verpackung zu einem Öffnen durch Aufreißen in Längsrichtung längs einer vorbestimmten Aufreißbahn ausgebildet ist.

Derartige Flow-Pack-Verpackungen sind allgemein bekannt und werden zur Verpackung mit einer Vielzahl von Produkten verwendet, etwa von Lebensmitteln oder aber auch von medizinischen Geräten. Letztere müssen insbesondere steril verpackt sein, wobei die Sterilisierung durch Erwärmung auf etwa 130 °C oder/und durch Bestrahlung mit Gammastrahlen erfolgen kann.

Mit "Flow-Pack-Verpackung" ist lediglich die Verpackungsform mit einem durch eine Längssiegelnaht erzeugten Folienlaminatschlauch bezeichnet, welcher an seinen beiden Längsenden durch je eine Quersiegelnaht verschlossen ist. Dementsprechend ist mit dem Begriff "Flow-Pack-Verpackung" in der vorliegenden Anmeldung auch eine sogenannte "Stick-Pack-Verpackung" bezeichnet, die eine Untergruppe der "Flow-Pack-Verpackung" bildet, bei welcher das Verhältnis der Länge der Längssiegelnaht zur Länge der Quersiegelnähte besonders groß ist, die also deutlich länger als breit sind. In der Technik unterscheiden sich Flow-Pack-Verpackungen und Stick-Pack-Verpackungen auch durch die Art ihrer Befüllung. So werden Flow-Pack-Verpackungen üblicherweise in horizontaler Lage geformt, befüllt und versiegelt, sogenannte "Horizontal Form Fill Seal (HFFS)", während Stick-Pack-Verpackungen üblicherweise in vertikaler Orientierung geformt, befüllt und versiegelt werden, sogenannte "Vertical Form Fill Seal (VFFS)". Auf die Orientierung eines Folienlaminats bei der Bildung der Verpackung, ihrer Befüllung und ihrem Versiegeln soll es zur Beurteilung, ob eine Flow-Pack-Verpackung vorliegt oder nicht, in der vorliegenden Anmeldung nicht ankommen.

Zur Abschirmung des Verpackungsraumes und des darin aufgenommenen Gutes vor äußeren Einflüssen weist das Folienlaminat, das zur Bildung der Flow-Pack-Verpackung verwendet wird, eine Barrierelage auf, die häufig in Form einer Metalllage, insbesondere Aluminiumlage, ausgebildet ist. Diese ist dann blickdicht, weshalb die bezogen auf den Verpackungsraum weiter außen gelegene äußere Lage als informationsträger über den Inhalt herangezogen wird. Denn nur eine bezüglich des Verpackungsraums weiter außen als die Barrierelage gelegene Lage kann überhaupt von einem Verbraucher optisch erfasst werden.

Die heißsiegelfähige Innenlage stellt die zur Bildung der Flow-Pack-Verpackung auf üblichen Verpackungsmaschinen notwendige Siegeleigenschaft der Innenlage mit sich selbst her.

Gerade dann, wenn mit der Flow-Pack-Verpackung medizinische Geräte oder Gegenstände verpackt werden sollen, ist es wichtig, dass deren Öffnen diskret erfolgen kann. Dies kann beispielsweise dadurch erreicht werden, dass die beim Aufreißen einer Verpackung entstehende Geräuschentwicklung gemindert wird. Eine derartige "leise" aufreißbare Flow-Pack-Verpackung ist jedoch auch in anderen Bereichen wünschenswert, etwa für Konsumverpackungen, die in öffentlichen Räumen benutzt werden sollen, wie etwa in Kinosälen.

Darüber hinaus soll die Flow-Pack-Verpackung effizient, d. h. in möglichst großen Stückzahlen pro Zeiteinheit, herstellbar sein.

Im Stand der Technik bekannte Flow-Pack-Verpackungen zur Verpackung von medizinischen Geräten sind durch Einsiegeln oder Ansiegeln eines Aufreißstreifens an bzw. in das zur Herstellung der Flow-Pack-Verpackung verwendete Folienlaminat zu einem Öffnen durch Aufreißen in Längsrichtung ausgebildet. Der Aufreißstreifen, in der Regel ein Kunststoffstreifen, welcher aus einem mit der heißsiegelfähigen Innenlage kompatiblen Material gebildet ist, wird hierzu derart an das Folienlaminat angesiegelt bzw. in dieses eingesiegelt, dass er in der fertigen Verpackung in deren Längsrichtung verläuft. Ein in die Flow-Pack-Verpackung des Standes der Technik eingebrachter Riss wird dann durch Abreißen des Aufreißstreifens der Verlaufsrichtung des Aufreißstreifens in an sich bekannter Weise folgen.

Das Vorsehen eines zusätzlichen Aufreißstreifens ist mit zusätzlichem Aufwand und somit mit zusätzlichen Kosten verbunden. Dies ist offensichtlich nachteilig.

Es ist die Aufgabe der vorliegenden Erfindung, die eingangs genannte Flow-Pack-Verpackung derart weiterzubilden, dass die diskret aufreißbar und mit möglichst geringem Aufwand in großer Stückzahl pro Zeiteinheit herstellbar ist.

Diese Aufgabe wird erfindungsgemäß gelöst durch eine gattungsgemäße Flow-Pack-Verpackung, bei welcher die äußere Lage aus monoaxial orientiertem Polyolefin gebildet ist, dessen Orientierungsrichtung in Längsrichtung der Verpackung verläuft, und die siegelfähige Innenlage ein Polyolefin umfasst, vorzugsweise aus einem Polyolefin gebildet ist.

Durch die monoaxiale Orientierung der äußeren Lage aus Polyolefin, welche insofern eine "äußere" Lage ist, als dass sie bezogen auf den Verpackungsraum weiter außen als die Barrierelage gelegen ist, kann das Folienlaminat, mit welchem die Flow-Pack-Verpackung gebildet wird, mit einer eindeutigen Aufreißrichtung versehen werden. Das Folienlaminat reißt dann längs der Orientierungsrichtung, jedoch nicht quer dazu. Grund hierfür ist die beim monoaxialen Orientieren erzielte Molekülausrichtung in der entsprechend orientierten Lage.

Ordnet man die Lage aus monoaxial orientiertem Polyolefin derart an, dass die Orientierungsrichtung dieser Lage an der fertiggestellten Flow-Pack-Verpackung in Längsrichtung verläuft, kann ein einmal zum Öffnen der Verpackung eingeleiteter Riss in Längsrichtung über die gesamte Länge der Flow-Pack-Verpackung ausgebreitet werden. Damit ist die Entnahme des verpackten Gutes besonders leicht möglich, da das Gut nicht nur durch eine bezogen auf eine Quer- oder eine Längsabmessung der Verpackung kleine Öffnung entnommen zu werden brauchen, sondern die Verpackung an sich vollständig längs ihrer gesamten Längsabmessung geöffnet ist und man mit der Hand in die Packung eingreifen kann. Dies ist besonders für ältere oder kranke Personen von Vorteil, die alters- oder krankheitsbedingt motorisch beeinträchtigt sind. Gerade derartige Personen benötigen jedoch häufig steril verpackte medizinische Vorrichtungen.

Die Verwendung von monoaxial orientiertem Polyolefin führt zu einer geringen Geräuschentwicklung beim Aufreißen. Dabei ist monoaxial orientiertes Polypropylen aufgrund seiner besonders geringen Aufreißgeräusche bevorzugt. Jedoch soll nicht ausgeschlossen sein, dass auch monoaxial orientiertes Polyethylen als Material der äußeren Lage verwendet wird. Auch dieses lässt sich verhältnismäßig geräuscharm aufreißen.

Durch Ausbildung der siegelfähigen Innenlage unter Beteiligung eines Polyolefins, vorzugsweise durch Bildung der Innenlage mit einem Polyolefin, können die Siegelnähte zur Herstellung der Verpackung bereits bei niedrigen Temperaturen erzeugt werden, so dass ein geringerer Energieeintrag in das Folienlaminatmaterial notwendig ist, um die Siegelnähte zu erzeugen, was wiederum zu einer verhältnismäßig kurzen Aufheizzeit führt. In der Folge können Siegelnähte mit siegelfähigen Innenlagen aus einem Polyolefin in rascherer Abfolge hergestellt werden als mit anderen Materialien. Eine besonders niedrige Siegeltemperatur bietet Polyethylen (PE), welches daher als das oben genannte Polyolefin bevorzugt ist. Aber auch andere Polyolefine, wie etwa Polypropylen, sollen in der Verwendung nicht ausgeschlossen sein.

Um sicherzustellen, dass das Folienlaminat der hier diskutierten Verpackung in dem Sinne gut siegelbar ist, dass bei Anlegen einer Wärmequelle einer Siegeleinrichtung an die Außenseite der Verpackung nur die siegelfähige Innenlage ausreichend erweicht, sind bevorzugt die Materialien der äußeren Lage und der Innenlage derart gewählt, dass der Schmelzindex (MFI = "Melt Flow Index") der äußeren Lage um wenigstens 10 % bezogen auf den Wert der Innenlage niedriger ist als der Schmelzindex der Innenlage.

Grundsätzlich soll nicht ausgeschlossen sein, dass das Folienlaminat zur Bildung der hier vorgestellten Flow-Pack-Verpackung noch jenseits der äußeren Lage weiter außen gelegene Lagen aufweist, bevorzugt ist jedoch vorgesehen, dass die äußere Lage die äußerste Lage des Folienlaminats ist. Besonders bevorzugt ist aus Kosten- und Gewichtsgründen jedoch ein Folienlaminat, welches - abgesehen von Druckauftrag und etwaig notwendigen haftvermittelten Schichten zwischen den Laminatlagen - mit den drei genannten Laminatlagen auskommt nur diese aufweist.

Wie oben bereits dargelegt wurde, bezeichnet in der vorliegenden Anmeldung der Begriff "Flow-Pack-Verpackung" lediglich die Gestalt der Verpackung, ohne Rücksicht auf die Orientierung des Folienlaminats bei der Bildung der Verpackung, bei deren Befüllung oder bei deren Versiegeln. Dementsprechend sind in der vorliegenden Anmeldung vom Begriff "Flow-Pack-Verpackung" ausdrücklich sogenannte "Stick-Pack-Verpackungen" umfasst, welche aus Gestaltgesichtspunkten Flow-Pack-Verpackungen mit besonders hohem Längen-zu-Breiten-Verhältnis sind. Grundsätzlich kann die hier diskutierte Verpackung sowohl im Verfahren des "Horizontal Form Fill Seal" als auch im Verfahren des "Vertical Form Fill Seal" marktfertig hergestellt werden, wenngleich für die besonders bevorzugte Anwendung der Verpackung von Kathetern, Spritzen und vergleichbaren medizinischen Geräten das Verfahren des "Vertical Form Fill Seal" bevorzugt ist.

Um sicherzugehen, dass ein einmal in die Flow-Pack-Verpackung eingeleiteter Riss sich entlang der Orientierungsrichtung der Lage aus monoaxial orientiertem Polyolefin (MOPO), insbesondere aus monoaxial orientiertem Polypropylen (MOPP), ausbreitet und nicht in seiner Ausbreitung durch die übrigen Lagen, insbesondere die siegelfähige Innenlage, gestört wird, kann vorgesehen sein, dass die Dicke der siegelfähigen Innenlage nicht mehr als das Doppelte der Dicke der monoaxial orientierten Polyolefinlage, insbesondere Polypropylenlage, beträgt, wobei vorzugsweise die Dicke der siegelfä-, higen Innenlage nicht kleiner als die Dicke der monoaxial orientierten Polyolefinlage, insbesondere Polypropylenlage, ist.

Das nachfolgend für eine MOPO-Lage Gesagte gilt insbesondere für eine MOPP-Lage, so dass "MOPO" im nachfolgenden Text durch "MOPP" ersetzt werden kann.

Mit der bisher verfügbaren Technologie zur Bedruckung von Lagen aus MOPO hat sich herausgestellt, dass die Dicke der MOPO-Lage wenigstens 20 µm betragen sollte, um einen qualitativ ausreichenden Druckauftrag darauf bereitzustellen. Deshalb sollte die Dicke der MOPO-Lage nicht weniger als 20 µm betragen. Falls diese nicht bedruckt wird, kann ihre Dicke jedoch geringer sein.

Um eine einfache Aufreißbarkeit auch durch geschwächte Personen sicherstellen zu können, sollte die Dicke der MOPO-Lage 100 µm nicht überschreiten. Ein guter Kompromiss zwischen Bedruckbarkeit mit guter Druckbildqualität und gleichzeitig leichter Aufreißbarkeit kann dann erhalten werden, wenn die Dicke der MOPO-Lage zwischen 25 µm und 50 µm beträgt. Versuche haben gezeigt, dass MOPO-Lagen mit Dicken zwischen 30 µm und 40 µm, wobei die angegebenen Grenzen explizit dem genannten bevorzugten Dickenbereich angehören, ein besonders leises Aufreißen auch über lange Strecken bis zu einem halben Meter gewährleisten können. Somit können mit MOPO-Lagen der genannten bevorzugten Dicken auch lange Flow-Pack-Verpackungen über ihre gesamte Länge leise aufgerissen werden.

Die Dicke der siegelfähigen Lage beträgt bevorzugt mehr als 25 µm, um die gewünschte Dichtigkeit der Flow-Pack-Verpackung zu gewährleisten. Eine Dicke von mehr als 50 µm der siegelfähigen Innenlage ist zwar grundsätzlich möglich, jedoch zur Erzielung einer sicheren, dauerhaften und dichten Siegelnaht nicht erforderlich. Daher sollte aus Gewichts- und Kostengründen die siegelfähige Innenlage eine Dicke von 50 µm nicht überschreiten. In praktischen Versuchen haben sich Dicken der siegelfähigen Innenlage im Bereich von 40 µm bis 50 µm als besonders vorteilhaft zur Erzielung dauerhafter dichter Siegelnähte erwiesen.

Um die gewünschte Rissführungsfähigkeit der äußeren Lage beim Aufreißen der Flow-Pack-Verpackung sicherzustellen, sollte das Reckungsverhältnis der äußeren Lage in einem Bereich von 1:2,5 bis 1:15, bevorzugt in einem Bereich von 1:2,5 bis 1:9, liegen. Dadurch wird eine ausreichende molekulare Orientierung der Moleküle in der äußeren Lage erreicht, um einen einmal in die äußere Lage eingeleiteten Riss längs der Orientierungsrichtung zu führen. Ein Reckverhältnis von 1:x bedeutet dabei, dass bei der Herstellung der Folie diese in einer Richtung, üblicherweise die Maschinenrichtung, um das x-fache gedehnt wird, während die Folie in ihrer zur Reckrichtung orthogonalen Erstreckungsrichtung, üblicherweise die Maschinenquerrichtung, in der Ausgangsabmessung verbleibt.

Das Folienlaminat zur Bildung der hier diskutierten Flow-Pack-Verpackung kann dann besonders vorteilhaft auf schon bereits eingerichteten Maschinen verarbeitet werden, wenn die Innenlage nicht orientiert ist, bzw. wenn nur die Außenlage eine Orientierung aufweist. Hierdurch wird zusätzlich eine vorteilhafte Funktionstrennung dahingehend erreicht, dass die äußere Lage möglichst gut die ihr zugedachte Funktion der Rissführung erfüllen kann, während die Innenlage möglichst gut und sicher mit sich selbst gesiegelt werden kann.

Sofern aus produktionstechnischen Gründen eine Orientierung auch der Innenlage nicht vermeidbar sein sollte, sollte deren Reckungsverhältnis jedoch nicht größer als 1:5 sein, besonders bevorzugt nicht größer als 1:2,5.

Die Barrierelage kann grundsätzlich ein beliebiges Material mit Sauerstoffbarriereeigenschaften sein, wie etwa EVOH. Bevorzugt wird jedoch eine Metalllage als Barrierelage verwendet, die besonders dünn ausgebildet werden kann, etwa im einstelligen µm-Bereich. Beispielsweise kann die Barrierelage in an sich bekannter Weise als Aluminiumlage ausgebildet sein. Eine derartige Aluminiumlage kann eine bevorzugte Dicke von 5 µm bis 12 µm, insbesondere von 7 µm bis 9 µm, aufweisen und somit die Rissführung der MOPO-Lage nicht beeinträchtigen und gleichzeitig eine ausreichende Sauerstoffbarriere bereitstellen.

Um zu gewährleisten, dass insbesondere durch Alter oder/und Krankheit geschwächte Personen die Flow-Pack-Verpackung öffnen können, kann diese eine Aufreißhilfe aufweisen. Die Aufreißhilfe kann als Materialschwächung ausgestaltet sein, beispielsweise in Form einer Prägung, einer Perforation, einer Schlitzung und dergleichen, von einer oder mehreren Lagen des Folienlaminats.

Mit der Aufreißhilfe soll zunächst ein Riss in die Verpackung eingeleitet werden, welcher nach seiner Einleitung durch die Orientierung der MOPO-Lage in Längsrichtung der Verpackung fortgesetzt werden soll. Um ein verfrühtes Öffnen der Verpackung zu vermeiden und damit zu verhindern, dass das verpackte Gut, welches insbesondere ein medizinisches Gerät, wie etwa ein Katheter, sein kann, beim Öffnen der Flow-Pack-Verpackung bereits aus dieser heraus fällt und unerwünschterweise vor einem Gebrauch desselben bereits verschmutzt, ist es vorteilhaft, wenn die Aufreißhilfe in einer der Quersiegelnähte verläuft, um im Bereich eines der Längsenden der Flow-Pack-Verpackung zu beginnen, und näher an einem Längsrand der Quersiegelnaht endet als am jeweils anderen, in Querrichtung entgegengesetzten Längsrand der Quernaht. Somit wird durch die Aufreißhilfe also ein Riss bis zu einer Stelle in der Quersiegelnaht eingeleitet, welcher näher an einem Längsrand als an dem jeweils anderen Längsrand gelegen ist, so dass ein von dort sich weiter in Längsrichtung der Verpackung ausbreitender Riss die Verpackung nicht in gleiche Hälften teilt. Die größere der verbleibenden Verpackungsabschnitte kann somit leicht zusammen mit dem noch verpackten Gut in einer Hand gehalten werden oder kann zumindest das verpackte Gut umgebend verbleiben. Vorzugsweise liegt die Aufreißhilfe vollständig in einer Quersiegelnaht.

Hier hat es sich als vorteilhaft erwiesen, wenn das Ende der Aufreißhilfe, welches schließlich den Beginn des die Verpackung öffnenden Risses in Längsrichtung der Flow-Pack-Verpackung markiert, die Querabmessung der sie aufweisenden Quersiegelnaht wenigstens im Verhältnis 1 : 2 teilt, also das Ende der Aufreißhilfe von dem ferneren Längsrand der Quersiegelnaht wenigstens zwei mal so weit entfernt gelegen ist, wie von dem jeweils anderen, näheren Längsrand.

Um ein sicheres Aufreißverhalten möglichst über die gesamte Länge der Verpackung zu erhalten, ist es vorteilhaft, wenn das Ende der Aufreißhilfe von dem ferneren Längsrand der Quersiegelnaht nicht mehr als 40 mal so weit entfernt gelegen ist, wie von dem näheren Längsrand. Je nach Abmessung der Quersiegelnaht und auch der Flow-Pack-Verpackung insgesamt in Querrichtung ist es weiter bevorzugt, wenn das Ende der Aufreißhilfe von dem ferneren Längsrand der Quersiegelnaht etwa 4 bis 30 mal, besonders bevorzugt etwa 5 bis 20 mal, so weit entfernt gelegen ist wie von dem näheren Längsrand.

Grundsätzlich kann die Aufreißhilfe in beliebiger Richtung zu dem genannten Endpunkt verlaufen, wobei jedoch unterschiedliche Gestaltungen zu einer unterschiedlich guten Handhabbarkeit, insbesondere durch motorisch beeinträchtigte Personen, führt. Dann, wenn die Aufreißhilfe längs einer Einreißrichtung verläuft, welche mit der Richtung der monoaxialen Orientierung der MOPO-Lage einen Winkel einschließt, kann erreicht werden, dass beiderseits der Aufreißhilfe ein ausreichend großes Verpackungsstück zum Handangriff für ein Öffnen bereitgestellt ist. Beispielsweise kann der Winkel 60° bis 120°, besonders bevorzugt 60° bis 80°, betragen.

Die Anwendung der oben genannten bevorzugten Winkelbereiche führt in der Regel dazu, dass die Aufreißhilfe an jenem Längsrand beginnt, die ihrem Ende in Querrichtung ferner liegt. Vorteilhafterweise ist die die Aufreißhilfe tragende Quersiegelnaht mit einer größeren Länge in Längsrichtung ausgebildet als die jeweils andere Quersiegelnaht, welche keine Aufreißhilfe zu tragen braucht.

Damit das Aufreißen der Verpackung, welches schließlich die wenigstens abschnittsweise Trennung von zwei ursprünglich miteinander verbundenen Verpackungsabschnitten umfasst, auch von geschwächten oder motorisch beeinträchtigten Personen erreicht werden kann, ist es hilfreich, wenn die Flow-Pack-Verpackung Angreifhilfen aufweist. Bevorzugt verläuft die Aufreißhilfe zwischen zwei Angreifhilfen hindurch, so dass ein Handangriff mit unterschiedlichen Händen an jede der Angreifhilfen dazu führt, dass genau jene Abschnitte der Flow-Pack-Verpackung gegriffen sind, die beim Aufreißen wenigstens abschnittsweise voneinander getrennt werden sollen. Die Angreifhilfen können besonders einfach aber wirksam in Form von Löchern gebildet sein, durch welche Finger oder Gegenstände, wie etwa Kugelschreiber oder Bleistifte, hindurchgesteckt werden können, um Kraft auf die die jeweiligen Angreifhilfen tragenden Verpackungsabschnitte auszuüben.

Vorzugsweise ist die MOPO-Lage im Konterdruckverfahren bedruckt, so dass sie selbst den Aufdruck vor Beschädigung schützt. Zwischen dem Aufdruck auf die MOPO-Lage und die daran anschließende Barrierelage kann ein Flaftvermittler aufgetragen sein. Dies gilt ebenso für die mit der Barrielelage verbundene siegelfähige Innenlage.

Gemäß einem bevorzugten Gesichtspunkt betrifft die vorliegende Erfindung eine Flow-Pack-Verpackung, wie sie oben beschrieben wurde, mit einem darin verpackten Katheter.

Die vorliegende Erfindung wird nachfolgend anhand der beiliegenden Zeichnungen näher erläutert. Es stellt dar:
- Fig. 1: einen Querschnitt durch ein Folienlaminat, aus welchem eine erfindungsgemäße Ausführungsform einer Flow-Pack-Verpackung gebildet sein kann, und
- Fig. 2: eine erfindungsgemäße Ausführungsform einer Flow-Pack-Verpackung.

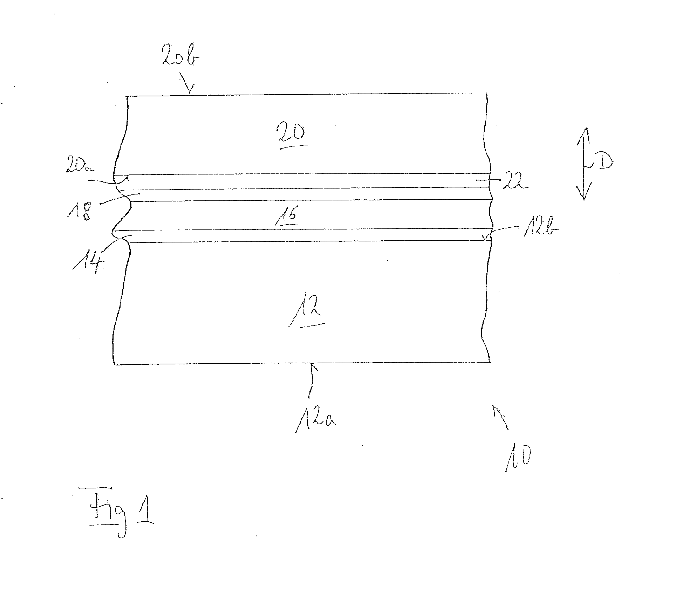

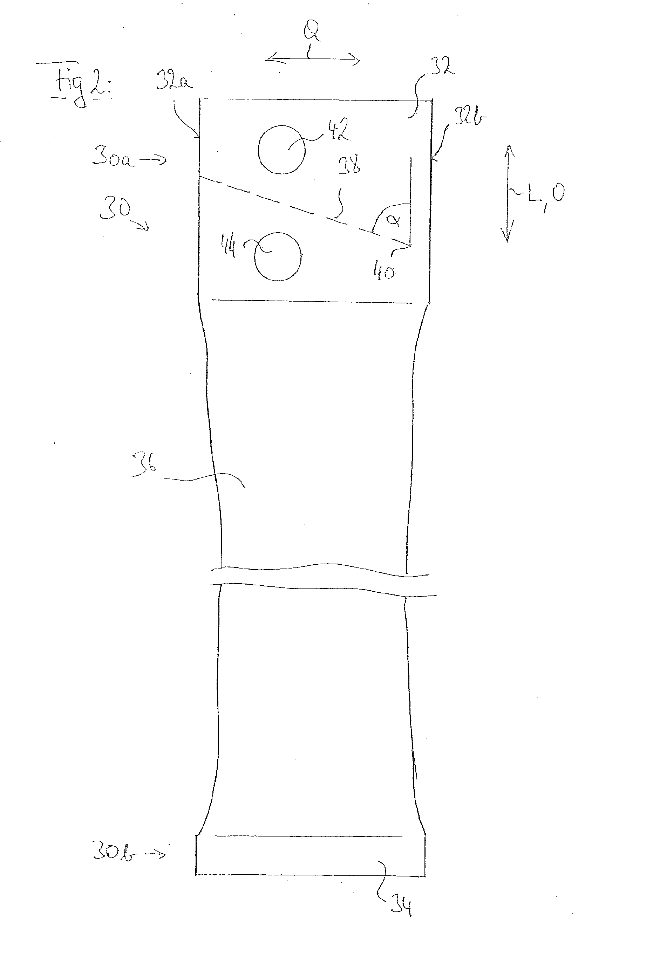

In Fig. 1 ist ein Folienlaminat zur Herstellung einer erfindungsgemäßen Ausführungsform einer Flow-Pack-Verpackung, wie sie in Fig. 2 gezeigt ist, allgemein mit 10 bezeichnet.

Das Folienlaminat 10 weist eine Innenlage 12 auf, welche eine innerste Lage ist und welche siegelfähig ist. Diese siegelfähige Innenlage 12 umfasst ein Polyolefin oder ist vorzugsweise aus einem Polyolefin gebildet, um eine möglichst niedrige Siegeltemperatur bereitstellen zu können. Das Polyolefin ist aufgrund seiner niedrigen Siegeltemperatur bevorzugt Polyethylen.

Die siegelfähige Innenlage 12 weist eine zum Verpackungsraum hin weisende und frei liegende Innenfläche 12a und eine nach außen weisende entgegengesetzte Fläche 12b auf.

Auf die Fläche 12b kann, falls erforderlich, eine dünne Haftmittelschicht 14 aufgetragen sein, mittels welcher die siegelfähige Innenlage 12 mit einer Barrierelage 16, etwa einer Aluminiumlage, verbunden sein kann.

Die Barrierelage 16, welche einen Sauerstoffdurchgang in Dickenrichtung D durch das Folienlaminat 10 verhindert oder wenigstens stark reduziert, kann über eine weitere Haftmittelschicht 18 mit einer äußeren Lage 20 aus monoaxial orientiertem Polyolefin verbunden sein, wobei im dargestellten Beispiel monoaxial orientiertes Polypropylen (MOPP) gewählt ist. Die MOPP-Lage 20 kann im Konterdruckverfahren mit einem Druckauftrag 22 bedruckt sein, um diesen vor äußeren Einflüssen zu schützen. Alternativ kann der Druckauftrag 22 anstatt auf die in Richtung des Verpackungsraums weisende Fläche 20a auf die nach außen weisende und frei liegende Fläche 20b der MOPP-Lage aufgetragen sein. Dann kann über den Druckauftrag ein Überlack aufgetragen sein, um den Druckauftrag vor mechanischer oder sonstiger Belastung zu schützen.

Die äußere Lage 20 aus monoaxial orientiertem Polypropylen (wobei statt des Polypropylens auch ein monoaxial orientiertes Polyethylen verwendet werden kann) weist im vorliegenden Fall eine monoaxiale Reckung mit einem Reckverhältnis im Bereich von 1:2,5 bis 1:15 auf, etwa ein Reckverhältnis von 1:8. Um sicherzustellen, dass bei einem Siegeln zwar von der Außenseite des Folienlaminats 10 her Wärme eingetragen werden kann, jedoch durch die eingetragene Wärme die Innenlage 12 erweicht und nicht die üblicherweise beim Siegeln näher an der Wärmequelle gelegene äußere Lage 20, sind die zur Bildung der äußeren Lage 20 und der Innenlage 12 verwendeten Materialien vorzugsweise so gewählt, dass der Schmelzindex des Materials der äußeren Lage 20, bezogen auf den Schmelzindex des Materials der Innenlage 12, um wenigstens 10 % geringer ist als der Schmelzindex des Materials der Innenlage 12.

Bei dem dargestellten bevorzugten Ausführungsbeispiel ist die äußere Lage 20 die einzige Lage, welche eine Reckung aufweist. Die Innenlage 12 ist bevorzugt, abgesehen von verfahrenstechnisch unvermeidlichen Reckwirkungen, gänzlich ungereckt.

In Fig. 2 ist eine erfindungsgemäße Ausführungsform einer Flow-Pack-Verpackung der vorliegenden Anmeldung in der Draufsicht dargestellt und mit 30 bezeichnet. Die Flow-Pack-Verpackung 30 ist in Längsrichtung L wesentlich länger ausgebildet als in ihrer Querrichtung Q.

An ihren Längsendbereichen 30a und 30b weist die Flow-Pack-Verpackung 30 jeweils eine Quersiegelnaht auf, wobei die Quersiegelnaht 32 am Längsende 30a in Längsrichtung L größer ausgebildet ist als die Quersiegelnaht 34 am Längsende 30b der Flow-Pack-Verpackung 30.

Eine sich im Wesentlichen in Längsrichtung L erstreckende Längssiegelnaht verläuft auf der Rückseite der Flow-Pack-Verpackung 30 und ist in Fig. 2 nicht dargestellt. In Fig. 2 ist ihre Vorderseite in Draufsicht dargestellt. In allen Siegelnähten der Flow-Pack-Verpackung sind vor dem Siegeln frei liegende Bereiche der siegelfähigen Innenlage 12 aneinander gesiegelt.

In Längsrichtung L zwischen den längsendseitigen Quersiegelnähten 32 und 34 ist ein Verpackungsraum 36 von der Flow-Pack-Verpackung 30 umschlossen, wobei in diesem Bereich das Folienlaminats 10 aufgrund der in Fig. 2 nicht gezeigten Längssiegelnaht einen schlauchartigen Abschnitt aufweist, welcher den Verpackungsraum 36 umgibt.

Die monoaxial orientierte Polypropylenlage 20, welche in dem dargestellten Beispiel der Flow-Pack-Verpackung 30 ganz außen liegt, ist derart orientiert, dass ihre Orientierungsrichtung O mit der Längsrichtung L der Flow-Pack-Verpackung 30 übereinstimmt. Risse breiten sich in der MOPP-Lage 20 im Wesentlichen längs der Orientierungsrichtung aus, so dass ein in die MOPP-Lage 20 eingebrachter Riss ohne weitere Maßnahmen sich in Längsrichtung L der Flow-Pack-Verpackung 30 ausbreiten wird.

Durch das gewählte Dickenverhältnis der äußeren MOPP-Lage 20 und der siegelfähigen Innenlage 12, wonach die Siegelfähige Innenlage nicht mehr als doppelt so dick ist wie die MOPP-Lage 20, vorzugsweise auch nicht dünner ist als diese, ist sichergestellt, dass die MOPP-Lage 20 dem gesamten Folienlaminat 10 ihr Rissausbreitungsverhalten aufzwingt.

Die Dicke der Barrierelage 16 beträgt im dargestellten Beispiel etwa 9 µm, während die MOPP-Lage 20 vorzugsweise 30 µm und die siegelfähige Innenlage 12 etwa 50 µm Dicke aufweist.

In einem alternativen Ausführungsbeispiel kann bei ansonsten unveränderter Barrierelage 16 die Dicke der MOPP-Lage und der siegelfähigen Innenlage im Wesentlichen jeweils 40 µm betragen.

In der längeren Quersiegelnaht 32 kann eine Aufreißhilfe 38 ausgebildet sein, etwa als Perforation, Schlitzung oder sonstige Materialschwächung des Folienlaminats 10. Eine derartig ausgebildete Materialschwächung kann das Aufreißverhalten der MOPP-Lage 20 stärker beeinflussen als deren Molekülorientierung durch monoaxiale Orientierung. Daher kann ein Riss, welcher von dem Längsrand 32a aus in die Quersiegelnaht 32 eingebracht wird, sich zunächst längs der Aufreißhilfe 38 ausbreiten. Diese erstreckt sich also vorzugsweise ausgehend von dem Längsrand 32a zu dem jeweils in Querrichtung Q entgegengesetzten anderen Längsrand 32b hin, wobei sie diesen nicht erreicht, um ein bloßes Durchtrennen der Quersiegelnaht 32 zu vermeiden. Vielmehr endet die Aufreißhilfe 38 an einem dem Längsrand 32b der Quersiegelnaht 32 näher gelegenen Endpunkt 40, von welchem aus sich ein zunächst längs einer durch die Aufreißhilfe 38 aufgezwungenen Aufreißrichtung in die Quersiegelnaht 32 und damit in die Verpackung 30 eingebrachter Riss weiter in Längsrichtung L ausbreitet und somit ein Aufreißen der Verpackung 30 über ihre im Wesentlichen gesamte Länge ermöglicht.

Der Endpunkt 40 der Aufreißhilfe 38 liegt im dargestellten Beispiel in Querrichtung Q von dem Längsrand 32a, an welchem die Aufreißhilfe 38 beginnt, etwa 9 mal so weit entfernt wie von dem Längsrand 32b. Durch diese Ausgestaltung kann 90 % der Verpackungsbreite weiter genutzt werden, um den verpackten Gegenstand, beispielsweise einen in Fig. 2 nicht dargestellten Katheter, im Verpackungsraum 36 trotz des Aufreißens größtenteils vom Verpackungsmaterial der Flow-Pack-Verpackung 30 umhüllt zu halten und dessen unerwünschte Verschmutzung trotz eines Öffnens der Flow-Pack-Verpackung 30 weitestgehend zu vermeiden.

Vorzugsweise schließt die Verlaufsrichtung der Aufreißhilfe 38 mit der Längsrichtung L der Flow-Pack-Verpackung einen Winkel α ein, welcher bevorzugt in einem Bereich von 60° bis 80° gelegen ist, im vorliegenden Beispiel sind es etwa 73°. Durch die Wahl eines Winkels von weniger als 90° weist der längs der Aufreißhilfe 38 in die Verpackung 30 eingebrachte Riss bereits eine Verlaufskomponente in Längsrichtung L der Flow-Pack-Verpackung 30 auf, so dass dessen weitere Ausbreitung am Endpunkt 40 der Aufreißhilfe 38 ausschließlich in Längsrichtung bzw. dessen Umtenkung in Richtung der Materialorientierung der MOPP-Lage 20 unterstützt wird. Von dem dargestellten Winkel α abweichende Winkel sind jedoch nicht ausgeschlossen.

Vorzugsweise verläuft die Aufreißhilfe 38 zwischen zwei Angreifhilfen 42 und 44 hindurch, von welchen jede mit einer anderen Hand eines Benutzers gegriffen werden kann. Dadurch ist es auch gebrechlicheren Personen möglich, ausreichend Kraft in die Quersiegelnaht 32 einzuleiten, um eine Trennung der Quersiegelnaht 32 zunächst längs der Aufreißhilfe 38 und ab deren Endpunkt 40 längs der Längsrichtung L einzuleiten. Zur bestmöglichen Krafteinleitung sind die Angreifhilfen 42 und 44 beide vorzugsweise vollständig in der die Aufreißhilfe 38 tragende Quersiegelnaht 32 eingebracht.

Besonders einfach, effektiv und zugleich gewichtssparend können die Angreifhilfen 42 und 44 durch Durchgangsöffnungen gebildet sein, vorzugsweise mit einem Durchmesser, der ein Durchstecken eines Fingers einer Hand oder wenigstens eines Stiftes, etwa eines Kugelschreibers oder eines Bleistiftes, ermöglicht. Dadurch wird auch schwächteren und motorisch beeinträchtigten Personen ein sicheres Öffnen der Flow-Pack-Verpackung 30 ermöglicht.

Überraschend an der hier vorgestellten Flow-Pack-Verpackung 30 ist, dass der abweichend von der Materialorientierung des Folienlaminats 10 über die Aufreißhilfe 38 in die Quersiegelnaht 32 eingetragene Riss an dem in der Quersiegelnaht 32 gelegenen Endpunkt 40 trotz der großen Nähe zu einem Längsrand (hier: Längsrand 32b) problemlos ohne weitere Maßnahme in die Längsrichtung L umgelenkt wird. Hierfür alleine ausschlaggebend ist die monoaxiale Orientierung der MOPP-Lage und die oben bezeichneten Dickenverhältnisse der am Folienlaminat 10 beteiligten Kunststofflagen.

## Patentansprüche

1. Flow-Pack-Verpackung (30) aus einem Folienlaminatstück, insbesondere zur Verpackung von medizinischen Geräten, bevorzugt von Kathetern, mit einer in Längsrichtung (L) verlaufenden Längssiegelnaht, welche entgegengesetzte Ränder des Folienlaminatstücks zu einem Folienlaminatschlauch verbindet, und mit je einer in einer zur Längsrichtung (L) orthogonalen Querrichtung (Q) verlaufenden Quersiegelnaht (32, 34) an jedem ihrer Längsenden (30a, 30b), welche Quersiegelnähte (32, 34) den Folienlaminatschlauch an seinen beiden Längsenden (30a, 30b) unter Bildung eines in Längsrichtung (L) zwischen den Quersiegelnähten (32, 34) gelegenen, vom Folienlaminatschlauch umgebenen Verpackungsraums (36) verschließen, wobei das Folienlaminat (10) eine bedruckbare äußere Lage (20), eine Barrierelage (16) und eine heißsiegelfähige Innenlage (12) umfasst, wobei die Flow-Pack-Verpackung (30) zu einem Öffnen durch Aufreißen in Längsrichtung (L) längs einer vorbestimmten Aufreißbahn ausgebildet ist,
**dadurch gekennzeichnet, dass** die äußere Lage (20) aus monoaxial orientiertem Polyolefin, insbesondere monoaxial gerecktem Polypropylen, dessen Orientierungsrichtung (O) in Längsrichtung (L) der Verpackung (30) verläuft, gebildet ist und die siegelfähige Innenlage (12) ein Polyolefin umfasst, vorzugsweise aus einem Polyolefin gebildet ist.

2. Flow-Pack-Verpackung nach Anspruch 1,
**dadurch gekennzeichnet, dass** das monoaxial orientierte Polyolefin der äußeren Lage, bezogen auf den Schmelzindex des Polyolefins der Innenlage, einen um wenigstens 10 % kleineren Schmelzindex aufweist.

3. Flow-Pack-Verpackung nach Anspruch 1 oder 2,
**dadurch gekennzeichnet, dass** die Dicke der siegelfähigen Innenlage (12) nicht mehr als das Doppelte der Dicke der monoaxial orientierten Polyolefinlage (20), insbesondere Polypropylenlage (20), beträgt, wobei vorzugsweise die Dicke der siegelfähigen Innenlage (12) nicht kleiner als die Dicke der monoaxial orientierten Polyolefinlage (20) ist.

4. Flow-Pack-Verpackung nach einem der vorhergehenden Ansprüche,
**dadurch gekennzeichnet, dass** die Dicke der monoaxial orientierten Polyolefinlage (20), insbesondere Polypropylenlage (20), nicht weniger als 20 µm beträgt, vorzugsweise zwischen 25 und 100 µm beträgt, besonders bevorzugt zwischen 25 µm und 50µm beträgt.

5. Flow-Pack-Verpackung nach Anspruch 4,
**dadurch gekennzeichnet, dass** die Dicke der monoaxial orientierten Polyolefinlage (20), insbesondere Polypropylenlage (20), zwischen 30 µm und 40 µm beträgt.

6. Flow-Pack-Verpackung nach einem der vorhergehenden Ansprüche,
**dadurch gekennzeichnet, dass** die Dicke der siegelfähigen Innenfage (12) zwischen 25 µm und 50 µm beträgt, bevorzugt zwischen 40 µm und 50 µm beträgt.

7. Flow-Pack-Verpackung nach einem der vorhergehenden Ansprüche,
**dadurch gekennzeichnet, dass** die äußere Lage (20) mit einem Reckungsverhältnis von 1:2,5 bis 1:15, bevorzugt von 1:2,5 bis 1:9, monoaxial gereckt ist.

8. Flow-Pack-Verpackung nach einem der vorhergehenden Ansprüche,
**dadurch gekennzeichnet, dass** die Innenlage (12) nicht gereckt ist, wobei bevorzugt die äußere Lage (20) die einzige Lage des Folienlaminats mit Reckung ist.

9. Flow-Pack-Verpackung nach einem der vorhergehenden Ansprüche,
**dadurch gekennzeichnet, dass** die Barrierelage (16) eine Metalllage, insbesondere eine Aluminiumlage ist.

10. Flow-Pack-Verpackung nach einem der vorhergehenden Ansprüche,
**dadurch gekennzeichnet, dass** sie eine Aufreißhilfe (38) aufweist, welche in einer der Quersiegelnähte (32) verläuft und näher an einem Längsrand (32b) der Quersiegelnaht (32) endet als am jeweils anderen, in Querrichtung (Q) entgegengesetzten Längsrand (32a) der Quersiegelnaht (32).

11. Flow-Pack-Verpackung nach Anspruch 10,
**dadurch gekennzeichnet, dass** das Ende (40) der Aufreißhilfe (38) von dem ferneren Längsrand (32a) der Quersiegelnaht (32) etwa 2 bis 40 mal, bevorzugt etwa 4 bis 30 mal, besonders bevorzugt etwa 5 bis 20 mal soweit entfernt gelegen ist wie von dem jeweils anderen, näheren Längsrand (32b).

12. Flow-Pack-Verpackung nach Anspruch 10 oder 11,
**dadurch gekennzeichnet, dass** die Aufreißhilfe (38) längs einer Einreißrichtung verläuft, welche mit der Richtung (O) der monoaxialen Orientierung der monoaxial orientierten Polyolefinlage (20) einen Winkel (α) einschließt, vorzugsweise im Bereich von 60° bis 120°, besonders bevorzugt im Bereich von 60° bis 80°.

13. Flow-Pack-Verpackung nach Anspruch 12,
**dadurch gekennzeichnet, dass** die Aufreißhilfe (38) an jenem Längsrand (32a) beginnt, der ihrem Ende (40) in Querrichtung (Q) ferner liegt.

14. Flow-Pack-Verpackung nach einem der Ansprüche 10 bis 13,
**dadurch gekennzeichnet, dass** die Aufreißhilfe (38) zwischen zwei Angreifhilfen (42, 44), vorzugsweise in Form von Löchern, hindurch verläuft.

15. Flow-Pack-Verpackung nach einem der vorhergehenden Ansprüche,
**dadurch gekennzeichnet, dass** die äußere Lage (20) im Konterdruckverfahren bedruckt ist.

16. Flow-Pack-Verpackung nach einem der vorhergehenden Ansprüche mit einem darin verpackten Katheter.

17. Flow-Pack-Verpackung nach einem der vorhergehenden Ansprüche,
**dadurch gekennzeichnet, dass** das Polyolefin der siegelfähigen Innenlage (12) Polyethylen ist.
